# EUROPEAN PATENT APPLICATION

(11) **EP 4 674 947 A1**
(43) Date of publication of application: **07.01.2026**
(21) Application number: 24875670.2
(22) Date of filing: 24.10.2024
(51) Int. Cl.: C12N 1/19

(54) **YEAST MUTANT STRAIN WITH HIGH GLYCOLIPID PRODUCTIVITY**

(30) Priority: 22.05.2024 WO PCT/JP2024/018870
(71) Applicant: KAO CORPORATION, Tokyo 103-8210 (JP)
(72) Inventor: KANEDA, Jitsuro, Tokyo 1038210 (JP); TAKAHASHI, Fumikazu, Tokyo 1038210 (JP)
(74) Representative: Jones, Nicholas Andrew
(86) International application number: PCT/JP2024/037996
(87) International publication number: WO 2025/243562

(57) **Abstract**

Provided is a yeast mutant strain having high glycolipid productivity. The yeast mutant strain, in which expression of a polypeptide consisting of the amino acid sequence of SEQ ID NO: 2 or an amino acid sequence having at least 90% identity to the amino acid sequence of SEQ ID NO: 2 is suppressed or the polypeptide is inactivated.

## Description

### Field of the Invention

The present invention relates to a mutant strain having high glycolipid productivity and a method for producing a glycolipid using the mutant strain.

### Background of the Invention

Sophorolipid is a glycolipid which is composed of sophorose linked to a long-chain hydroxy fatty acid and produced by microorganisms, mainly, yeasts. Sophorolipid is an amphipathic lipid having strong surface activity and excellent biodegradability. Therefore, its application as a biosurfactant has received attention in recent years. Sophorolipid has good skin affinity because of being a microbic product and being composed mainly of a nonionic component, and as such, is used as a permeation enhancer for cosmetics. Furthermore, sophorolipid is excellent in biodegradability and effective even when added in a small amount, and as such, is increasingly used in the field of cleansing agents such as dishwashing detergents.

*Starmerella bombicola* [former *Candida bombicola*]*, a* nonpathogenic basidiomycetous yeast, is well known as a yeast which produces sophorolipid. Sophorolipid produced by *Starmerella bombicola* has a lactone-type or acid-type structure, exhibits a critical micelle concentration of from 40 to 100 mg/L, and decreases a surface tension of water from 72.8 mN/m to 30 mN/N (Non Patent Literature 1). Sophorolipid differs in physicochemical properties due to different structures thereof. It has been reported that properties such as antimicrobial properties or surface activity vary between the lactone type and the acid type or among different fatty acid species constituting the sophorolipid (Non Patent Literatures 1 and 2).

Sophorolipid, when used as a cleansing agent or cosmetic material, is forced to compete with surfactants currently used. General surfactants have heretofore been produced at very low cost because of being bulk chemicals. Thus, there is a strong demand for reduction in sophorolipid production cost.

Study and improvement have heretofore been made mainly on yields, purification methods, techniques of conferring foaming properties, and the like for sophorolipid production processes (Patent Literatures 1 and 2). Also, methods for improving sophorolipid productivity by modifying a specific gene of *Starmerella bombicola* have been reported (Patent Literatures 3 and 4). A novel approach of improving sophorolipid productivity is required for further reducing cost.

(Patent Literature 1) JP-A-2003-9896
(Patent Literature 2) JP-A-2014-150774
(Patent Literature 3) JP-B-6563721
(Patent Literature 4) JP-B-6725506

(Non Patent Literature 1) Appl Microbiol Biotech, 2007, 76(1): 23-34
(Non Patent Literature 2) J SURFACT DETERG, 2006, 9, QTR 1: 57-62

### Summary of the Invention

The present invention relates to the following 1) to 4).
1) A yeast mutant strain in which expression of a polypeptide consisting of the amino acid sequence of SEQ ID NO: 2 or an amino acid sequence having at least 90% identity to the amino acid sequence of SEQ ID NO: 2 is suppressed or the polypeptide is inactivated.
2) A method for producing a yeast mutant strain, comprising suppressing expression of or inactivating a polypeptide consisting of the amino acid sequence of SEQ ID NO: 2 or an amino acid sequence having at least 90% identity to the amino acid sequence of SEQ ID NO: 2 in a yeast.
3) A method for improving ability of a yeast to produce a glycolipid, comprising suppressing expression of or inactivating a polypeptide consisting of the amino acid sequence of SEQ ID NO: 2 or an amino acid sequence having at least 90% identity to the amino acid sequence of SEQ ID NO: 2 in the yeast.
4) A method for producing a glycolipid, comprising culturing the yeast mutant strain according to 1).

### Detailed Description of the Invention

The present invention relates to provision of a yeast mutant strain which can produce a glycolipid with high efficiency, and a method for producing a glycolipid using the same.

The present inventors found that a yeast in which a polypeptide consisting of expression of the amino acid sequence of SEQ ID NO: 2 is suppressed or the polypeptide is inactivated improves its ability to produce a glycolipid.

The present invention provides a yeast mutant strain having high ability to produce a glycolipid. The yeast mutant strain of the present invention can efficiently produce a glycolipid.

All patent and non-patent literatures and other publications cited herein are hereby incorporated by reference in their entirety.

### (1. Definition)

In the present specification, the identity of an amino acid sequence or a nucleotide sequence is calculated by the Lipman-Pearson method (Science, 1985, 227: 1435-1441). Specifically, the identity is calculated by analysis using the search homology program of genetic information processing software GENETYX Ver. 12 with the unit size to compare (ktup) set to 2.

In the present specification, the term "at least 90% identity" regarding an amino acid sequence or a nucleotide sequence refers to 90% or more, preferably 95% or more, more preferably 97% or more, further more preferably 98% or more, further more preferably 99% or more identity.

In the present specification, the term "amino acid sequence obtained by deletion, substitution, addition, or insertion of one or several amino acids" refers to an amino acid sequence obtained by deletion, substitution, addition, or insertion of 1 or more and 75 or less, preferably 1 or more and 40 or less, more preferably 1 or more and 20 or less, further more preferably 1 or more and 10 or less, even more preferably 1 or more and 8 or less, even more preferably 1 or more and 5 or less, even more preferably 1 or more and 3 or less amino acids. The term "nucleotide sequence obtained by deletion, substitution, addition, or insertion of one or several nucleotides" refers to a nucleotide sequence derived by the deletion, substitution, addition, or insertion of 1 or more and 220 or less, preferably 1 or more and 120 or less, more preferably 1 or more and 60 or less, further more preferably 1 or more and 30 or less, even more preferably 1 or more and 24 or less, even more preferably 1 or more and 15 or less, even more preferably 1 or more and 9 or less nucleotides. In the present specification, the "addition" of an amino acid or a nucleotide includes the addition of the amino acid or the nucleotide to one end and both ends of a sequence.

In the present specification, the terms "upstream" and "downstream" regarding a gene refer to upstream and downstream in the transcriptional direction of the gene. For example, the term "gene located downstream of a promoter" means that the gene resides on the 3' side of the promoter in a DNA sense strand, and the term "upstream of a gene" means a region on the 5' side of the gene in a DNA sense strand.

In the present specification, the term "yeast" refers to a glycolipid-producing yeast, and the "glycolipid-producing yeast" refers to a yeast having the ability to produce a glycolipid. Among them, a sophorolipid-producing yeast is preferred. Examples of such a yeast include *Ascomycetes* such as the genus *Starmerella,* the genus *Candida,* and the genus *Wickerhamiella.* A yeast of the genus *Starmerella* is preferred. Examples of the yeast of the genus *Starmerella* include *Starmerella bombicola.* Examples of the yeast of the genus *Candida* include *Candida bogoriensis, Candida batistae,* and *Candida apicola.* Examples of the yeast of the genus *Wickerhamiella* include *Wickerhamiella domericqiae.* More preferred examples thereof include *Starmerella bombicola.*

The polypeptide whose expression is suppressed or which is inactivated in the yeast mutant strain of the present invention is a polypeptide consisting of the amino acid sequence of SEQ ID NO: 2 or a polypeptide equivalent thereto. The amino acid sequence of SEQ ID NO: 2 is an amino acid sequence based on specific ORF found as a result of conducting ORF analysis by the present inventors on *Starmerella bombicola* which has not previously been sufficiently studied by ORF analysis. As shown in Examples described later, the yeast mutant strain in which a gene encoding a polypeptide consisting of the amino acid sequence of SEQ ID NO: 2 is deleted has the improved ability to produce a glycolipid. Accordingly, the polypeptide consisting of the amino acid sequence of SEQ ID NO: 2 is presumed to be a polypeptide involved in the ability of a yeast to produce a glycolipid. Suppressed expression or inactivation of the polypeptide improves the ability of a yeast to produce a glycolipid. As a result of BLAST search of National Center for Biotechnology Information (NCBI), a protein having high sequence identity to the polypeptide consisting of the amino acid sequence of SEQ ID NO: 2 has been a transcriptional repressor called TUP1 derived from *Sugiyamaella lignohabitans,* but the sequence identity therebetween was as low as 62.9%. Hence, the polypeptide consisting of the amino acid sequence of SEQ ID NO: 2 has been considered as a previously unknown novel protein and designated as b0790 protein.

In the present specification, the "polypeptide equivalent to the polypeptide consisting of the amino acid sequence of SEQ ID NO: 2" is a polypeptide consisting of an amino acid sequence having at least 90% identity to the amino acid sequence of SEQ ID NO: 2. Examples of the amino acid sequence having at least 90% identity to the amino acid sequence of SEQ ID NO: 2 include an amino acid sequence obtained by deletion, substitution, addition, or insertion of one or several amino acids in the amino acid sequence of SEQ ID NO: 2. The polypeptide equivalent to the polypeptide consisting of the amino acid sequence of SEQ ID NO: 2 is a polypeptide which improves the ability of a yeast to produce a glycolipid by suppressed expression or inactivation.

In the present specification, the term "gene encoding a polypeptide consisting of the amino acid sequence of SEQ ID NO: 2, i.e., b0790 gene" is preferably a gene consisting of the nucleotide sequence of SEQ ID NO: 1.

In the present specification, the term "gene encoding a polypeptide equivalent to a polypeptide consisting of the amino acid sequence of SEQ ID NO: 2" is a gene having the same function as that of the b0790 gene and is preferably a gene which consists of a nucleotide sequence having at least 90% identity to the nucleotide sequence of SEQ ID NO: 1 and encodes the polypeptide consisting of the amino acid sequence of SEQ ID NO: 2 or a polypeptide equivalent thereto. Examples of a nucleotide sequence having at least 90% identity to the nucleotide sequence of SEQ ID NO: 1 include a nucleotide sequence obtained by deletion, substitution, addition, or insertion of one or several nucleotides in the nucleotide sequence of SEQ ID NO: 1.

### (2. Yeast mutant strain)

The present invention provides a yeast mutant strain (hereinafter, referred to as the mutant strain of the present invention). In the mutant strain of the present invention, a polypeptide consisting of expression of the amino acid sequence of SEQ ID NO: 2 or a polypeptide equivalent thereto is suppressed or the polypeptide is inactivated. Preferably, the mutant strain of the present invention is a mutant strain produced by suppressing expression of or inactivating a polypeptide consisting of the amino acid sequence of SEQ ID NO: 2 or a polypeptide equivalent thereto by artificial modification in a yeast.

Preferably, the mutant strain of the present invention is a mutant strain in which expression of a polypeptide consisting of the amino acid sequence of SEQ ID NO: 2 or a polypeptide equivalent thereto is suppressed as compared with a strain before mutation (parent strain). In one embodiment, the mutant strain of the present invention can be a mutant strain in which an expression level of a polypeptide consisting of the amino acid sequence of SEQ ID NO: 2 or a polypeptide equivalent thereto is decreased to 50% or less, preferably 40% or less, more preferably 30% or less, further more preferably 20% or less, even more preferably 10% or less, further preferably 5% or less as compared with the parent strain. Still further preferably, the mutant strain of the present invention can be a mutant strain in which an expression level of a polypeptide consisting of the amino acid sequence of SEQ ID NO: 2 or a polypeptide equivalent thereto is lost to an undetectable level (equal to or less than a negative control or background expression level). The expression level of a polypeptide can be measured by a protein expression quantification method usually used, for example, but not limited to, a colorimetry method, a fluorescent method, Western blotting, ELISA, or radioimmunoassay.

The mutant strain of the present invention has the improved ability to produce a glycolipid by suppressed expression or inactivation of a polypeptide consisting of the amino acid sequence of SEQ ID NO: 2 or a polypeptide equivalent thereto. In this context, the "improved ability to produce a glycolipid" means that the amount of a glycolipid produced in the mutant strain of the present invention is improved as compared with a strain before mutation (parent strain). The "glycolipid" refers to a glycolipid containing glucose or an acetylated form thereof as a sugar or a sugar constituting a sugar chain. Examples of glycolipid include preferably sophorolipid, Bola sophoroside, and/or alkyl sophoroside, more preferably sophorolipid and/or Bola sophoroside. The amount of a glycolipid produced by the mutant strain of the present invention having the improved ability to produce a glycolipid is improved to preferably 105% or more, more preferably 110% or more, further more preferably 120% or more, even more preferably 130% or more, as compared with the amount of a glycolipid produced by the parent strain. In the present specification, the ability to produce a glycolipid is preferably based on the amount of a glycolipid produced after culture for 20 to 200 hours, more preferably for 50 to 200 hours.

Examples of the approach of suppressing expression of or inactivating a polypeptide consisting of the amino acid sequence of SEQ ID NO: 2 or a polypeptide equivalent thereto include a method of deleting or inactivating a gene encoding the polypeptide, a method of inactivating mRNA transcribed from a gene encoding the polypeptide, a method of suppressing translation of mRNA of a gene encoding the polypeptide by RNA interference or the like using siRNA, a method of mutating a gene encoding the polypeptide to reduce activity of the polypeptide, and a method of inactivating the polypeptide by using an inhibitory substance such as an aptamer or an antibody. In a preferred embodiment, the mutant strain of the present invention is a mutant strain in which a gene encoding the polypeptide consisting of the amino acid sequence of SEQ ID NO: 2 or a gene equivalent thereto is deleted or inactivated.

Examples of the approach of deleting or inactivating a gene of a yeast cell include mutagenesis (deletion, insertion, substitution, or addition) of one or more nucleotides on the nucleotide sequence of the target gene, substitution of the nucleotide sequence with another nucleotide sequence, insertion of another nucleotide sequence to the nucleotide sequence, and partial or whole deletion of the nucleotide sequence. Alternatively, similar mutagenesis or substitution, insertion, or deletion of a nucleotide sequence may be performed for a control region such as a promoter region of the target gene. For example, the promoter activity of a promoter which controls the expression of the target gene is reduced or eliminated by mutagenesis or replacement with a low-expression promoter so that transcription of mRNA from the target gene can be reduced or eliminated to inactivate the target gene.

A method for genetic engineering of a microorganism known in the art can be used as a specific approach for the mutagenesis or substitution, insertion, or deletion of a nucleotide sequence. Examples of the method can include, but are not limited to, ultraviolet irradiation, site-directed mutagenesis, homologous recombination using SOE-PCR (splicing by overlap extension PCR: Gene, 1989, 77: 61-68), and genome editing using artificial DNA nuclease or programmable nuclease.

After the mutagenesis or substitution, insertion, or deletion of a nucleotide sequence, the mutant strain of the present invention can be obtained by selecting a cell having the desired mutation by gene analysis or evaluation of an expression level of mRNA or a polypeptide encoded by the target gene.

Alternatively, when the approach of deleting or inactivating the gene is homologous recombination using SOE-PCR, a mutant strain lacking the target gene can be obtained by incorporating a drug resistance marker gene into a DNA fragment for gene deletion with which DNA of the target gene is substituted, culturing cells into which the DNA fragment for deletion has been introduced in a medium containing a drug, and separating a grown colony. Further, the mutation may be confirmed by the gene analysis or the evaluation of a polypeptide expression level mentioned above. These procedures can produce the yeast mutant strain of the present invention in which a gene encoding a polypeptide consisting of the amino acid sequence of SEQ ID NO: 2 or a gene equivalent thereto is deleted or inactivated.

Alternatively, the mutant strain of the present invention in which expression of a polypeptide consisting of the amino acid sequence of SEQ ID NO: 2 or a polypeptide equivalent thereto is suppressed or the polypeptide is inactivated may be obtained by confirming improvement in the ability to produce a glycolipid in the mutant strain prepared by the procedures described above.

### (3. Method for improving ability of mutant strain to produce glycolipid)

The mutant strain of the present invention produced by suppressed expression or inactivation of a polypeptide consisting of the amino acid sequence of SEQ ID NO: 2 or a polypeptide equivalent thereto mentioned above has the improved ability to produce a glycolipid as compared with a strain before mutation (parent strain). The present invention also provides a method for improving ability of a yeast to produce a glycolipid, including suppressing expression of or inactivating a polypeptide consisting of the amino acid sequence of SEQ ID NO: 2 or a polypeptide equivalent thereto in the yeast.

### (4. Method for producing glycolipid)

The mutant strain of the present invention has the improved ability to produce a glycolipid. Also, the mutant strain of the present invention can produce glycolipids with hydrocarbon chains, fatty acids, and the like having various chain lengths as substrates. Thus, the mutant strain of the present invention can be cultured together with a substrate having a proper chain length to efficiently produce a glycolipid containing a constituent hydrocarbon chain, fatty acid, or the like having the desired chain length. Thus, the present invention also provides a method for producing a glycolipid, including culturing the mutant strain of the present invention.

The glycolipid is not particularly limited as long as the glycolipid is capable of being produced by the mutant strain of the present invention. Examples thereof preferably include glycolipids containing glucose or an acetylated form thereof as a sugar or a sugar constituting a sugar chain, more preferably include glycolipids containing glucose, sophorose, cellobiose, or an acetylated form thereof as a sugar or a sugar chain, further more preferably include sophorolipid, Bola sophorolipid, Bola sophoroside, alkyl sophoroside, alkyl glucoside, Bola glucoside, acidic glucolipid, and/or cellobiose lipid, and even more preferably include sophorolipid, Bola sophoroside, and/or alkyl sophoroside, still further more preferably sophorolipid and/or Bola sophoroside.

The transformant for use in the method for producing a glycolipid according to the present invention is a yeast mutant strain in which expression of a polypeptide consisting of the amino acid sequence of SEQ ID NO: 2 or an amino acid sequence having at least 90% identity to the amino acid sequence of SEQ ID NO: 2 is suppressed or the polypeptide is inactivated, and may be subjected to further modification so as to improve the ability to produce glycolipids. Examples of the further modification include suppression of expression of or inactivation of a polypeptide involved in suppression of the ability to produce the glycolipid of interest, enhancement of expression of a polypeptide involved in improvement of the ability to produce the glycolipid of interest, and enhancement of expression of various enzymes for production of the glycolipid of interest.

Preferred examples of the further modification include suppression of expression of or inactivation of FAO1 (fatty alcohol oxidase 1) or a protein equivalent thereto. In this context, the "FAO1" is a polypeptide having oxidase activity for aliphatic alcohols, and is preferably FAO1 of *Starmerella bombicola,* more preferably a polypeptide consisting of the amino acid sequence of SEQ ID NO: 34. The "protein equivalent to the FAO1" is a protein having the same function as that of the FAO1 and includes a homolog or an ortholog of the FAO1, or a mutant thereof. The protein equivalent to the FAO1 is preferably a polypeptide consisting of an amino acid sequence having at least 90% identity to the amino acid sequence of SEQ ID NO: 34. Examples of the amino acid sequence having at least 90% identity to the amino acid sequence of SEQ ID NO: 34 include an amino acid sequence obtained by deletion, substitution, addition, or insertion of one or several amino acids in the amino acid sequence of SEQ ID NO: 34. The modification is preferably deletion or inactivation of a gene encoding the polypeptide consisting of the amino acid sequence of SEQ ID NO: 34 or an amino acid sequence having at least 90% identity to the amino acid sequence of SEQ ID NO: 34, more preferably deletion or inactivation of a gene consisting of the nucleotide sequence of SEQ ID NO: 33 or a gene consisting of a nucleotide sequence having at least 90% identity to the nucleotide sequence of SEQ ID NO: 33, further more preferably deletion or inactivation of a gene consisting of the nucleotide sequence of SEQ ID NO: 33 or a gene which consists of a nucleotide sequence having at least 90% identity to the nucleotide sequence of SEQ ID NO: 33 and encodes the polypeptide consisting of the amino acid sequence of SEQ ID NO: 34 or an amino acid sequence having at least 90% identity to the amino acid sequence of SEQ ID NO: 34. The polypeptide consisting of the amino acid sequence of SEQ ID NO: 34 is encoded by the nucleotide sequence of SEQ ID NO: 33 in *Starmerella bombicola,* and a *Starmerella bombicola* mutant strain in which expression of the polypeptide is suppressed or the polypeptide is inactivated has been reported to become capable of producing Bola sophoroside, alkyl sophoroside, alkyl glucoside, and Bola glucoside (Appl Microbiol Biotechnol. 2016 Nov; 100 (22): 9519-9528). Specific examples of the mutant stain in which expression of FAO1 or a protein equivalent thereto is suppressed or the FAO1 or the protein is inactivated include a *Starmerella bombicola* Δfao1Δb0790:hyg strain shown in Examples described later.

Preferred examples of the further modification include suppression of expression of or inactivation of a polypeptide consisting of the amino acid sequence of SEQ ID NO: 36 or an amino acid sequence having at least 90% identity to the amino acid sequence of SEQ ID NO: 36. The polypeptide consisting of the amino acid sequence of SEQ ID NO: 36 or an amino acid sequence having at least 90% identity to the amino acid sequence of SEQ ID NO: 36 is a putative transcription factor protein, more preferably a putative transcription factor protein having two zinc finger C2H2-type DNA binding domains. The presence or absence of the zinc finger C2H2-type DNA binding domain in a certain polypeptide can be confirmed, for example, by analyzing an amino acid sequence using CD search of NCBI, though the method for analyzing the presence or absence of the domain is not limited thereto. The modification is preferably deletion or inactivation of a gene encoding the polypeptide consisting of the amino acid sequence of SEQ ID NO: 36 or an amino acid sequence having at least 90% identity to the amino acid sequence of SEQ ID NO: 36, more preferably deletion or inactivation of a gene consisting of the nucleotide sequence of SEQ ID NO: 35 or a gene consisting of a nucleotide sequence having at least 90% identity to the nucleotide sequence of SEQ ID NO: 35, further more preferably deletion or inactivation of a gene consisting of the nucleotide sequence of SEQ ID NO: 35 or a gene which consists of a nucleotide sequence having at least 90% identity to the nucleotide sequence of SEQ ID NO: 35 and encodes the polypeptide consisting of the amino acid sequence of SEQ ID NO: 36 or an amino acid sequence having at least 90% identity to the amino acid sequence of SEQ ID NO: 36. The polypeptide consisting of the amino acid sequence of SEQ ID NO: 36 is encoded by the nucleotide sequence of SEQ ID NO: 35 in *Starmerella bombicola,* and a *Starmerella bombicola* mutant strain in which expression of the polypeptide is suppressed or the polypeptide is inactivated has been reported to improve the productivity of sophorolipids as compared with its parent strain (JP-B-6725506).

Preferred examples of the further modification include suppression of expression of or inactivation of CYP52M1 (CYP52M1 cytochrome P450 monooxygenase) or a protein equivalent thereto. In this context, the "CYP52M1" is a polypeptide having hydroxylation activity at ω or ω-1 position of fatty acid or aliphatic alcohol, and is preferably CYP52M1 of *Starmerella bombicola,* more preferably a polypeptide consisting of the amino acid sequence of SEQ ID NO: 38. The "protein equivalent to the CYP52M1" is a protein having the same function as that of the FAO1 and includes a homolog or an ortholog of the CYP52M1, or a mutant thereof. The protein equivalent to the CYP52M1 is preferably a polypeptide consisting of an amino acid sequence having at least 90% identity to the amino acid sequence of SEQ ID NO: 38. Examples of the amino acid sequence having at least 90% identity to the amino acid sequence of SEQ ID NO: 38 include an amino acid sequence obtained by deletion, substitution, addition, or insertion of one or several amino acids in the amino acid sequence of SEQ ID NO: 38. The modification is preferably deletion or inactivation of a gene encoding the polypeptide consisting of the amino acid sequence of SEQ ID NO: 38 or an amino acid sequence having at least 90% identity to the amino acid sequence of SEQ ID NO: 38, more preferably deletion or inactivation of a gene consisting of the nucleotide sequence of SEQ ID NO: 37 or a gene consisting of a nucleotide sequence having at least 90% identity to the nucleotide sequence of SEQ ID NO: 37, further more preferably deletion or inactivation of a gene consisting of the nucleotide sequence of SEQ ID NO: 37 or a gene which consists of a nucleotide sequence having at least 90% identity to the nucleotide sequence of SEQ ID NO: 37 and encodes the polypeptide consisting of the amino acid sequence of SEQ ID NO: 38 or an amino acid sequence having at least 90% identity to the amino acid sequence of SEQ ID NO: 38. The polypeptide consisting of the amino acid sequence of SEQ ID NO: 38 is encoded by the nucleotide sequence of SEQ ID NO: 37 in *Starmerella bombicola,* and a *Starmerella bombicola* mutant strain in which cyp52M1 gene and fao1 gene are disrupted has been reported to have good productivity of Bola sophoroside, and suppress by-production of sophorolipid (WO 2020/104582).

Preferred examples of the further modification include suppression of expression of or inactivation of CYP52N1 (CYP52N1 cytochrome P450 monooxygenase) or a protein equivalent thereto. In this context, the "CYP52N1" is, as in the CYP52M1, a polypeptide having hydroxylation activity at ω or ω-1 position of fatty acid or aliphatic alcohol (Inge N. A. Van Bogaert et al., FEMS Yeast Res., 2009, 9(1): 87-94), and is preferably CYP52N1 of *Starmerella bombicola,* more preferably a polypeptide consisting of the amino acid sequence of SEQ ID NO: 40. The "protein equivalent to the CYP52N1" is a protein having the same function as that of the CYP52N1 and includes a homolog or an ortholog of the CYP52N1, or a mutant thereof. The protein equivalent to the CYP52N1 is preferably a polypeptide consisting of an amino acid sequence having at least 90% identity to the amino acid sequence of SEQ ID NO: 40. Examples of the amino acid sequence having at least 90% identity to the amino acid sequence of SEQ ID NO: 40 include an amino acid sequence obtained by deletion, substitution, addition, or insertion of one or several amino acids in the amino acid sequence of SEQ ID NO: 40. The modification is preferably deletion or inactivation of a gene encoding the polypeptide consisting of the amino acid sequence of SEQ ID NO: 40 or an amino acid sequence having at least 90% identity to the amino acid sequence of SEQ ID NO: 40, more preferably deletion or inactivation of a gene consisting of the nucleotide sequence of SEQ ID NO: 39 or a gene consisting of a nucleotide sequence having at least 90% identity to the nucleotide sequence of SEQ ID NO: 39, further more preferably deletion or inactivation of a gene consisting of the nucleotide sequence of SEQ ID NO: 39 or a gene which consists of a nucleotide sequence having at least 90% identity to the nucleotide sequence of SEQ ID NO: 39 and encodes the polypeptide consisting of the amino acid sequence of SEQ ID NO: 40 or an amino acid sequence having at least 90% identity to the amino acid sequence of SEQ ID NO: 40. The polypeptide consisting of the amino acid sequence of SEQ ID NO: 40 is encoded by the nucleotide sequence of SEQ ID NO: 39 in *Starmerella bombicola.*

Preferred examples of the further modification include suppression of expression of or inactivation of CYP52E3 (CYP52E3 cytochrome P450 monooxygenase) or a protein equivalent thereto. In this context, the "CYP52E3" is, as in the CYP52M1, a polypeptide having hydroxylation activity at ω or ω-1 position of fatty acid or aliphatic alcohol (Inge N. A. Van Bogaert et al., FEMS Yeast Res., 2009, 9(1): 87-94), and is preferably CYP52E3 of *Starmerella bombicola,* more preferably a polypeptide consisting of the amino acid sequence of SEQ ID NO: 42. The "protein equivalent to the CYP52E3" is a protein having the same function as that of the CYP52E3 and includes a homolog or an ortholog of the CYP52E3, or a mutant thereof. The protein equivalent to the CYP52E3 is preferably a polypeptide consisting of an amino acid sequence having at least 90% identity to the amino acid sequence of SEQ ID NO: 42. Examples of the amino acid sequence having at least 90% identity to the amino acid sequence of SEQ ID NO: 42 include an amino acid sequence obtained by deletion, substitution, addition, or insertion of one or several amino acids in the amino acid sequence of SEQ ID NO: 42. The modification is preferably deletion or inactivation of a gene encoding the polypeptide consisting of the amino acid sequence of SEQ ID NO: 42 or an amino acid sequence having at least 90% identity to the amino acid sequence of SEQ ID NO: 42, more preferably deletion or inactivation of a gene consisting of the nucleotide sequence of SEQ ID NO: 41 or a gene consisting of a nucleotide sequence having at least 90% identity to the nucleotide sequence of SEQ ID NO: 41, further more preferably deletion or inactivation of a gene consisting of the nucleotide sequence of SEQ ID NO: 41 or a gene which consists of a nucleotide sequence having at least 90% identity to the nucleotide sequence of SEQ ID NO: 41 and encodes the polypeptide consisting of the amino acid sequence of SEQ ID NO: 42 or an amino acid sequence having at least 90% identity to the amino acid sequence of SEQ ID NO: 42. The polypeptide consisting of the amino acid sequence of SEQ ID NO: 42 is encoded by the nucleotide sequence of SEQ ID NO: 41 in *Starmerella bombicola.*

Preferred examples of the further modification include suppression of expression of or inactivation of UGTB1 (UDP-glucosyltransferase B1) or a protein equivalent thereto. In this context, the "UGTB1" is a polypeptide having transglycosylation activity to hydroxylated fatty acids from UDP-glucose as a donor, and is preferably UGTB1of *Starmerella bombicola,* more preferably a polypeptide consisting of the amino acid sequence of SEQ ID NO: 44. The "protein equivalent to the UGTB1" is a protein having the same function as that of the UGTB1 and includes a homolog or an ortholog of the UGTB1, or a mutant thereof. The protein equivalent to the UGTB1 is preferably a polypeptide consisting of an amino acid sequence having at least 90% identity to the amino acid sequence of SEQ ID NO: 44. Examples of the amino acid sequence having at least 90% identity to the amino acid sequence of SEQ ID NO: 44 include an amino acid sequence obtained by deletion, substitution, addition, or insertion of one or several amino acids in the amino acid sequence of SEQ ID NO: 44. The modification is preferably deletion or inactivation of a gene encoding the polypeptide consisting of the amino acid sequence of SEQ ID NO: 44 or an amino acid sequence having at least 90% identity to the amino acid sequence of SEQ ID NO: 44, more preferably deletion or inactivation of a gene consisting of the nucleotide sequence of SEQ ID NO: 43 or a gene consisting of a nucleotide sequence having at least 90% identity to the nucleotide sequence of SEQ ID NO: 43, further more preferably deletion or inactivation of a gene consisting of the nucleotide sequence of SEQ ID NO: 43 or a gene which consists of a nucleotide sequence having at least 90% identity to the nucleotide sequence of SEQ ID NO: 43 and encodes the polypeptide consisting of the amino acid sequence of SEQ ID NO: 44 or an amino acid sequence having at least 90% identity to the amino acid sequence of SEQ ID NO: 44. The polypeptide consisting of the amino acid sequence of SEQ ID NO: 44 is encoded by the nucleotide sequence of SEQ ID NO: 43 in *Starmerella bombicola.* A *Starmerella bombicola* mutant strain in which ugtB1 gene is disrupted has been reported to become capable of producing glucolipid (Sofie Lodens et al., Biotechnol Bioeng., 2020, 117(2): 453-465). Further, a *Starmerella bombicola* mutant strain in which ugtB1 gene, fao1 gene, and cyp52M1 gene are disrupted has been reported to become capable of producing alkyl glucoside and alkyl sophoroside (WO 2021/229017).

Preferred examples of the further modification include suppression of expression of or inactivation of UGTA1 (UDP-glucosyltransferase A1) or a protein equivalent thereto. In this context, the "UGTA1" is, as in the UGTB1, a polypeptide having transglycosylation activity to hydroxylated fatty acids from UDP-glucose as a donor (Karen M. J. Saerens et al., FEMS Yeast Res., 2015, 15(7): fov075), and is preferably UGTA1 of *Starmerella bombicola,* more preferably a polypeptide consisting of the amino acid sequence of SEQ ID NO: 46. The "protein equivalent to the UGTA1" is a protein having the same function as that of the UGTA1 and includes a homolog or an ortholog of the UGTA1, or a mutant thereof. The protein equivalent to the UGTA1 is preferably a polypeptide consisting of an amino acid sequence having at least 90% identity to the amino acid sequence of SEQ ID NO: 46. Examples of the amino acid sequence having at least 90% identity to the amino acid sequence of SEQ ID NO: 46 include an amino acid sequence obtained by deletion, substitution, addition, or insertion of one or several amino acids in the amino acid sequence of SEQ ID NO: 46. The modification is preferably deletion or inactivation of a gene encoding the polypeptide consisting of the amino acid sequence of SEQ ID NO: 46 or an amino acid sequence having at least 90% identity to the amino acid sequence of SEQ ID NO: 46, more preferably deletion or inactivation of a gene consisting of the nucleotide sequence of SEQ ID NO: 45 or a gene consisting of a nucleotide sequence having at least 90% identity to the nucleotide sequence of SEQ ID NO: 45, further more preferably deletion or inactivation of a gene consisting of the nucleotide sequence of SEQ ID NO: 45 or a gene which consists of a nucleotide sequence having at least 90% identity to the nucleotide sequence of SEQ ID NO: 45 and encodes the polypeptide consisting of the amino acid sequence of SEQ ID NO: 46 or an amino acid sequence having at least 90% identity to the amino acid sequence of SEQ ID NO: 46. The polypeptide consisting of the amino acid sequence of SEQ ID NO: 46 is encoded by the nucleotide sequence of SEQ ID NO: 45 in *Starmerella bombicola.*

Preferred examples of the further modification include suppression of expression of or inactivation of AT (acetyl transferase) or a protein equivalent thereto. In this context, the "AT" is a polypeptide having acetylation activity of OH moiety of sugar, and is preferably AT of *Starmerella bombicola,* more preferably a polypeptide consisting of the amino acid sequence of SEQ ID NO: 48. The "protein equivalent to the AT" is a protein having the same function as that of the AT and includes a homolog or an ortholog of the AT, or a mutant thereof. The protein equivalent to the AT is preferably a polypeptide consisting of an amino acid sequence having at least 90% identity to the amino acid sequence of SEQ ID NO: 48. Examples of the amino acid sequence having at least 90% identity to the amino acid sequence of SEQ ID NO: 48 include an amino acid sequence obtained by deletion, substitution, addition, or insertion of one or several amino acids in the amino acid sequence of SEQ ID NO: 48. The modification is preferably deletion or inactivation of a gene encoding the polypeptide consisting of the amino acid sequence of SEQ ID NO: 48 or an amino acid sequence having at least 90% identity to the amino acid sequence of SEQ ID NO: 48, more preferably deletion or inactivation of a gene consisting of the nucleotide sequence of SEQ ID NO: 47 or a gene consisting of a nucleotide sequence having at least 90% identity to the nucleotide sequence of SEQ ID NO: 47, further more preferably deletion or inactivation of a gene consisting of the nucleotide sequence of SEQ ID NO: 47 or a gene which consists of a nucleotide sequence having at least 90% identity to the nucleotide sequence of SEQ ID NO: 47 and encodes the polypeptide consisting of the amino acid sequence of SEQ ID NO: 48 or an amino acid sequence having at least 90% identity to the amino acid sequence of SEQ ID NO: 48. The polypeptide consisting of the amino acid sequence of SEQ ID NO: 48 is encoded by the nucleotide sequence of SEQ ID NO: 47 in *Starmerella bombicola.* A *Starmerella bombicola* mutant strain in which at gene is disrupted has been reported to produce non-acetylated sophorolipid (Karen M. J. Saerens et al., Biotechnol Bioeng., 2011, 108(12): 2923-2931).

Preferred examples of the further modification include suppression of expression of or inactivation of SBLE (*Starmerella Bombicola* Lactone Esterase) or a protein equivalent thereto. In this context, the "SBLE " is a polypeptide having lactonization activity of sophorolipid, and is preferably SBLE of *Starmerella bombicola,* more preferably a polypeptide consisting of the amino acid sequence of SEQ ID NO: 50. The "protein equivalent to the SBLE" is a protein having the same function as that of the SBLE and includes a homolog or an ortholog of the SBLE, or a mutant thereof. The protein equivalent to the SBLE is preferably a polypeptide consisting of an amino acid sequence having at least 90% identity to the amino acid sequence of SEQ ID NO: 50. Examples of the amino acid sequence having at least 90% identity to the amino acid sequence of SEQ ID NO: 50 include an amino acid sequence obtained by deletion, substitution, addition, or insertion of one or several amino acids in the amino acid sequence of SEQ ID NO: 50. The modification is preferably deletion or inactivation of a gene encoding the polypeptide consisting of the amino acid sequence of SEQ ID NO: 50 or an amino acid sequence having at least 90% identity to the amino acid sequence of SEQ ID NO: 50, more preferably deletion or inactivation of a gene consisting of the nucleotide sequence of SEQ ID NO: 49 or a gene consisting of a nucleotide sequence having at least 90% identity to the nucleotide sequence of SEQ ID NO: 49, further more preferably deletion or inactivation of a gene consisting of the nucleotide sequence of SEQ ID NO: 49 or a gene which consists of a nucleotide sequence having at least 90% identity to the nucleotide sequence of SEQ ID NO: 49 and encodes the polypeptide consisting of the amino acid sequence of SEQ ID NO: 50 or an amino acid sequence having at least 90% identity to the amino acid sequence of SEQ ID NO: 50. The polypeptide consisting of the amino acid sequence of SEQ ID NO: 50 is encoded by the nucleotide sequence of SEQ ID NO: 49 in *Starmerella bombicola.* A *Starmerella bombicola* mutant strain in which sble gene is disrupted has been reported to improve the percentage of acidic sophorolipid (Katarzyna Ciesielska et al., Appl Microbiol Biotechnol., 2016, 100(22): 9529-954). Further, a *Starmerella bombicola* mutant strain in which sble gene and at gene are disrupted has been reported to become capable of producing non-acetylated Bola sophorolipid (WO 2015/028278).

Preferred examples of the further modification include enhancement of expression of MDR1 (multidrug resistance protein 1) or a protein equivalent thereto. In this context, the "MDR1" is a transporter polypeptide responsible for intracellular and extracellular transport of sophorolipid, and is preferably MDR1 of *Starmerella bombicola,* more preferably a polypeptide consisting of the amino acid sequence of SEQ ID NO: 52. The "protein equivalent to the MDR1" is a protein having the same function as that of the MDR1 and includes a homolog or an ortholog of the MDR1, or a mutant thereof. The protein equivalent to the MDR1 is preferably a polypeptide consisting of an amino acid sequence having at least 90% identity to the amino acid sequence of SEQ ID NO: 52. Examples of the amino acid sequence having at least 90% identity to the amino acid sequence of SEQ ID NO: 52 include an amino acid sequence obtained by deletion, substitution, addition, or insertion of one or several amino acids in the amino acid sequence of SEQ ID NO: 52. The modification is preferably enhancement of expression of a gene encoding the polypeptide consisting of the amino acid sequence of SEQ ID NO: 52 or an amino acid sequence having at least 90% identity to the amino acid sequence of SEQ ID NO: 52, more preferably enhancement of expression of a gene consisting of the nucleotide sequence of SEQ ID NO: 51 or a gene consisting of a nucleotide sequence having at least 90% identity to the nucleotide sequence of SEQ ID NO: 51, further more preferably enhancement of expression of a gene consisting of the nucleotide sequence of SEQ ID NO: 51 or a gene which consists of a nucleotide sequence having at least 90% identity to the nucleotide sequence of SEQ ID NO: 51 and encodes the polypeptide consisting of the amino acid sequence of SEQ ID NO: 52 or an amino acid sequence having at least 90% identity to the amino acid sequence of SEQ ID NO: 52. The polypeptide consisting of the amino acid sequence of SEQ ID NO: 52 is encoded by the nucleotide sequence of SEQ ID NO: 51 in *Starmerella bombicola.* A *Starmerella bombicola* mutant strain in which mdr1 gene is disrupted has been reported to significantly reduce productivity of sophorolipid and Bola sophorolipid (Silke Claus et al., BMC Genomics, 2022, 23(1): 22).

Preferred examples of the further modification include suppression of expression of or inactivation of MFE-2 (multifunctional enzyme type 2) or a protein equivalent thereto. In this context, the "MFE-2" is a polypeptide involved in beta oxidative decomposition of fatty acid, and is preferably MFE-2 of *Starmerella bombicola.* The "protein equivalent to the MFE-2" is a protein having the same function as that of the MFE-2 and includes a homolog or an ortholog of the MFE-2, or a mutant thereof. A *Starmerella bombicola* mutant strain in which mfe-2 gene is disrupted has been reported to produce sophorolipid having a medium-chain fatty acid backbone (Inge N. A. Van Bogaert et al., FEMS Yeast Res., 2009, 9(4): 610-617).

In the method for producing a glycolipid according to the present invention, the mutant strain of the present invention is cultured in a medium containing a substrate such as fatty acid, fatty acid alkyl ester, triacylglycerol, diacylglycerol, monoacylglycerol, fat or oil, alkane, alkene, alkyne, and alcohol. The glycolipid can be recovered from the medium after culture and appropriately purified, if necessary, to produce a glycolipid.

A common medium containing a carbon source, a nitrogen source, an inorganic salt, and optionally an organic micronutrient such as an amino acid and a vitamin can be used as the medium for use in the culture. The medium may be any of a synthetic medium and a natural medium.

The carbon source and the nitrogen source contained in the medium may be of any type utilizable by the mutant strain to be cultured. Examples of the carbon source include: sugars such as glucose, glycerol, fructose, sucrose, maltose, mannose, galactose, starch hydrolysates, and molasses; organic acids such as acetic acid and citric acid; and alcohols such as ethanol. Any one of these carbon sources can be used alone, or two or more thereof can be used in combination. Examples of the nitrogen source include: ammonia; ammonium salts such as ammonium sulfate, ammonium carbonate, ammonium chloride, ammonium phosphate, and ammonium acetate; nitrate; and urea.

Examples of the inorganic salt include phosphate, magnesium salt, calcium salt, iron salt, and manganese salt. Examples of the organic micronutrient include amino acids, vitamins, fatty acids, nucleic acids, and peptone, casamino acid, yeast extracts, and soybean protein degradants containing these. In the case of using an auxotrophic mutant strain which requires an amino acid or the like for growth, the required nutrient may be added to the medium.

Preferred examples of the substrate which may be contained in the medium include C12 to 22 fatty acid and alkyl ester thereof, triacylglycerol, diacylglycerol, and monoacylglycerol containing C12 to 22 fatty acid or alkyl ester thereof, fat or oil containing C12 to 22 fatty acid or alkyl ester thereof, C12 to 22 alkane, C12 to 22 alkene, C12 to 22 alkyne, and C12 to 22 alcohol. More preferred examples thereof include C12 to 18 fatty acid and alkyl ester thereof, triacylglycerol, diacylglycerol, and monoacylglycerol containing C12 to C18 fatty acid or alkyl ester thereof, fat or oil containing C12 to C18 fatty acid or alkyl ester thereof, C12 to 18 alkane, C12 to 18 alkene, C12 to 18 alkyne, and C12 to 18 alcohol. Further more preferred examples thereof include C12 to C18 fatty acid and alkyl ester thereof, triacylglycerol containing C12 to C18 fatty acid or alkyl ester thereof, and C12 to C18 alcohol.

More detailed examples of the substrate include, but are not limited to: the C12 to 22 fatty acid, which may be saturated fatty acid or unsaturated fatty acid and may be linear fatty acid or branched fatty acid, including lauric acid, tridecylic acid, myristic acid, pentadecylic acid, palmitic acid, margaric acid, stearic acid, isostearic acid, nonadecylic acid, arachidic acid, behenic acid, palmitoleic acid, oleic acid, linolic acid, α-linolenic acid, γ-linolenic acid, arachidonic acid, eicosapentaenoic acid, and docosahexaenoic acid; the alkyl ester of the C12 to 22 fatty acid, including alkyl ester having 1 to 4 carbon atoms, preferably methyl ester and ethyl ester, of the fatty acid; and the fat or oil containing C12 to 22 fatty acid or alkyl ester thereof, including coconut oil, palm oil, palm kernel oil, olive oil, rapeseed oil, rice bran oil, soybean oil, castor oil, and madhuca oil.

Examples of the C12 to 22 alkane, which may be linear or branched, include dodecane, tridecane, tetradecane, pentadecane, hexadecane, heptadecane, octadecane, nonadecane, icosane, heneicosane, and docosane. Examples of the C12 to 22 alkene, which may be linear or branched, include 1-dodecene, 1-tridecene, 1-tetradecene, 1-pentadecene, 1-hexadecene, 1-heptadecene, 1-octadecene, 1-nonadecene, 1-icosene, 1-heneicosene, and 1-docosene. Examples of the C12 to 22 alkyne, which may be linear or branched, include 1-dodecyne, 1-tridecyne, 1-tetradecyne, 1-pentadecyne, 1-hexadecyne, 1-heptadecyne, 1-octadecyne, 1-nonadecyne, 1-icosyne, 1-heneicosyne, and 1-docosyne.

Examples of the C12 to 22 alcohol, which may be saturated alcohol or unsaturated alcohol and may be linear alcohol or branched alcohol, include lauryl alcohol, tridecyl alcohol, myristyl alcohol, pentadecyl alcohol, cetyl alcohol, heptadecyl alcohol, stearyl alcohol, isostearyl alcohol, nonadecyl alcohol, arachidyl alcohol, behenyl alcohol, palmitoleyl alcohol, oleyl alcohol, linoleyl alcohol, and linolenyl alcohol.

Any one of the substrates listed above can be used alone, or two or more thereof can be used in combination. Preferably, fatty acid having any chain length of from C12 to C18 or alkyl ester thereof, or triacylglycerol, diacylglycerol, monoacylglycerol, or fat or oil containing it, or alkane, alkene, alkyne, or alcohol having any chain length of from C12 to 18 is used. More preferably, fatty acid having any chain length of from C12 to C18 or alkyl ester thereof, or alcohol having any chain length of from C12 to 18 is used. Further more preferably, fatty acid having any chain length of from C16 to C18 or alkyl ester thereof, or alcohol having any chain length of from C16 to 18 is used. Even more preferably, C18 fatty acid or alkyl ester thereof, or C18 alcohol is used. Even more preferably, oleic acid or alkyl ester thereof, or oleyl alcohol is used. Rapeseed oil is also preferably used which is composed mainly of triacylglycerol rich in oleic acid as constituent fatty acid.

The content of the substrate (at the time of addition of the substrate) which may be contained in the medium is preferably 0.1 mass/volume % or more, more preferably 0.5 mass/volume% or more, further more preferably 1 mass/volume% or more, even more preferably mass/volume% or more, even more preferably 3 mass/volume% or more, even more preferably 5 mass/volume% or more, from a viewpoint of amount of glycolipid produced, and preferably 40 mass/volume% or less, more preferably 30 mass/volume% or less, further more preferably 25 mass/volume% or less , even more preferably 20 mass/volume% or less, even more preferably 15 mass/volume% or less, even more preferably 10 mass/volume% or less, from a viewpoint of glycolipid production efficiency. Alternatively, the content is preferably from 0.1 to 40 mass/volume%, from 0.1 to 30 mass/volume%, from 0.1 to 25 mass/volume%, from 0.1 to 20 mass/volume%, from 0.1 to 15 mass/volume%, from 0.1 to 10 mass/volume%, from 0.5 to 40 mass/volume%, from 0.5 to 30 mass/volume%, from 0.5 to 25 mass/volume%, from 0.5 to 20 mass/volume%, from 0.5 to 15 mass/volume%, from 0.5 to 10 mass/volume%, from 1 to 40 mass/volume%, from 1 to 30 mass/volume%, from 1 to 25 mass/volume%, from 1 to 20 mass/volume%, from 1 to 15 mass/volume%, from 1 to 10 mass/volume%, from 2 to 40 mass/volume%, from 2 to 30 mass/volume%, from 2 to 25 mass/volume%, from 2 to 20 mass/volume%, from 2 to 15 mass/volume%, from 2 to 10 mass/volume%, from 3 to 40 mass/volume%, from 3 to 30 mass/volume%, from 3 to 25 mass/volume%, from 3 to 20 mass/volume%, from 3 to 15 mass/volume%, from 3 to 10 mass/volume%, from 5 to 40 mass/volume%, from 5 to 30 mass/volume%, from 5 to 25 mass/volume%, from 5 to 20 mass/volume%, from 5 to 15 mass/volume%, or from 5 to 10 mass/volume%. In the present specification, the volume means a volume at 25°C and 1 atm.

The culture conditions may not be limited as long as the mutant strain of the present invention fermentatively produces a glycolipid under the conditions. The culture is preferably performed under aerobic conditions, and a general method such as aeration stirring culture or shake culture can be applied thereto. The culture temperature is preferably from 20 to 33°C, more preferably from 25 to 30°C, further more preferably from 28 to 30°C. The initial pH (30°C) of the medium is preferably from 2 to 7, more preferably from 3 to 6. The culture time is preferably about from 24 to 200 hours, more preferably from 50 to 200 hours.

In the culture, the mutant strain of the present invention may be cultured under cell proliferation conditions to fermentatively produce a glycolipid, or may be cultured in a dormant cell state, i.e., in a state where growth and proliferation are stopped, to fermentatively produce a glycolipid.

A method for recovering the glycolipid from the medium after culture is not particularly limited and can be performed in accordance with a recovery method known in the art. The glycolipid in the medium can be recovered or purified by using, for example, solvent extraction using ethyl acetate or butanol, fractional precipitation, liquid-liquid partition, column chromatography, and high-performance liquid chromatography alone or in appropriate combination.

The present specification further discloses a composition, a production method, application, or a method given below as an exemplary embodiment of the present invention. However, the present invention is not limited by these embodiments.
[1] A yeast mutant strain in which expression of a polypeptide consisting of the amino acid sequence of SEQ ID NO: 2 or an amino acid sequence having at least 90% identity to the amino acid sequence of SEQ ID NO: 2 is suppressed or the polypeptide is inactivated.
[2] The mutant strain according to [1], wherein a gene encoding the polypeptide consisting of the amino acid sequence of SEQ ID NO: 2 or an amino acid sequence having at least 90% identity to the amino acid sequence of SEQ ID NO: 2 is deleted or inactivated.
[3] The mutant strain according to [2], wherein a gene consisting of the nucleotide sequence of SEQ ID NO: 1 or a gene which consists of a nucleotide sequence having at least 90% identity to the nucleotide sequence of SEQ ID NO: 1 and encodes the polypeptide consisting of the amino acid sequence of SEQ ID NO: 2 or an amino acid sequence having at least 90% identity to the amino acid sequence of SEQ ID NO: 2 is deleted or inactivated.
[4] The mutant strain according to any of one of [1] to [3], in which expression of a polypeptide consisting of the amino acid sequence of SEQ ID NO: 34 or an amino acid sequence having at least 90% identity to the amino acid sequence of SEQ ID NO: 34 is suppressed or the polypeptide is inactivated.
[5] The mutant strain according to any one of [1] to [4], wherein the yeast is a glycolipid-producing yeast.
[6] The mutant strain according to any one of [1] to [5], wherein the yeast is a yeast of the genus *Starmerella,* preferably *Starmerella bombicola.*
[7] The mutant strain according to any one of [1] to [6], wherein the mutant strain has improved ability to produce a glycolipid as compared with a strain before mutation.
[8] The mutant strain according to any one of [5] to [7], wherein the glycolipid is selected from the group consisting of Bola sophorolipid, Bola sophoroside, alkyl sophoroside, alkyl glucoside, Bola glucoside, acidic glucolipid, and cellobiose lipid, preferably selected from the group consisting of sophorolipid, Bola sophoroside, and alkyl sophoroside, more preferably sophorolipid and Bola sophoroside.
[9] A method for producing a yeast mutant strain, comprising suppressing expression of or inactivating a polypeptide consisting of the amino acid sequence of SEQ ID NO: 2 or an amino acid sequence having at least 90% identity to the amino acid sequence of SEQ ID NO: 2 in a yeast.
[10] The method according to [9], comprising deleting or inactivating a gene encoding the polypeptide consisting of the amino acid sequence of SEQ ID NO: 2 or an amino acid sequence having at least 90% identity to the amino acid sequence of SEQ ID NO: 2.
[11] The method according to [10], comprising deleting or inactivating a gene consisting of the nucleotide sequence of SEQ ID NO: 1 or a gene which consists of a nucleotide sequence having at least 90% identity to the nucleotide sequence of SEQ ID NO: 1 and encodes the polypeptide consisting of the amino acid sequence of SEQ ID NO: 2 or an amino acid sequence having at least 90% identity to the amino acid sequence of SEQ ID NO: 2.
[12] The method according to any one of [9] to [11], further comprising suppressing expression of or inactivating a polypeptide consisting of the amino acid sequence of SEQ ID NO: 34 or an amino acid sequence having at least 90% identity to the amino acid sequence of SEQ ID NO: 34.
[13] The method according to any one of [9] to [12], wherein the yeast is a glycolipid-producing yeast.
[14] The method according to any one of [9] to [13], wherein the yeast is a yeast of the genus *Starmerella,* preferably *Starmerella bombicola.*
[15] The method according to any one of [9] to [14], wherein the mutant strain has improved ability to produce a glycolipid as compared with a strain before mutation.
[16] The method according to any one of [9] to [15], wherein the glycolipid is selected from the group consisting of Bola sophorolipid, Bola sophoroside, alkyl sophoroside, alkyl glucoside, Bola glucoside, acidic glucolipid, and cellobiose lipid, preferably selected from the group consisting of sophorolipid, Bola sophoroside, and alkyl sophoroside, more preferably sophorolipid and Bola sophoroside.
[17] A method for improving ability of a yeast to produce a glycolipid, comprising suppressing expression of or inactivating a polypeptide consisting of the amino acid sequence of SEQ ID NO: 2 or an amino acid sequence having at least 90% identity to the amino acid sequence of SEQ ID NO: 2 in the yeast.
[18] The method according to [17], comprising deleting or inactivating a gene encoding the polypeptide consisting of the amino acid sequence of SEQ ID NO: 2 or an amino acid sequence having at least 90% identity to the amino acid sequence of SEQ ID NO: 2.
[19] The method according to [18], comprising deleting or inactivating a gene consisting of the nucleotide sequence of SEQ ID NO: 1 or a gene which consists of a nucleotide sequence having at least 90% identity to the nucleotide sequence of SEQ ID NO: 1 and encodes the polypeptide consisting of the amino acid sequence of SEQ ID NO: 2 or an amino acid sequence having at least 90% identity to the amino acid sequence of SEQ ID NO: 2.
[20] The method according to any one of [17] to [19], further comprising suppressing expression of or inactivating a polypeptide consisting of the amino acid sequence of SEQ ID NO: 34 or an amino acid sequence having at least 90% identity to the amino acid sequence of SEQ ID NO: 34.
[21] The method according to any one of [17] to [20], wherein the yeast is a yeast of the genus *Starmerella,* preferably *Starmerella bombicola.*
[22] The method according to any one of [17] to [21], wherein the glycolipid is selected from the group consisting of Bola sophorolipid, Bola sophoroside, alkyl sophoroside, alkyl glucoside, Bola glucoside, acidic glucolipid, and cellobiose lipid, preferably selected from the group consisting of sophorolipid, Bola sophoroside, and alkyl sophoroside, more preferably sophorolipid and Bola sophoroside.
[23] A method for producing a glycolipid, comprising culturing the yeast mutant strain according to any one of [1] to [8].
[24] The method according to [23], wherein the glycolipid is selected from the group consisting of Bola sophorolipid, Bola sophoroside, alkyl sophoroside, alkyl glucoside, Bola glucoside, acidic glucolipid, and cellobiose lipid, preferably selected from the group consisting of sophorolipid, Bola sophoroside, and alkyl sophoroside, more preferably sophorolipid and Bola sophoroside.
[25] The method according to [23] or [24], wherein a medium for the culturing contains the following substrate:
   at least one substrate selected from the group consisting of C12 to C22 fatty acid and alkyl ester thereof, triacylglycerol, diacylglycerol, and monoacylglycerol containing C12 to C22 fatty acid or alkyl ester thereof, fat or oil containing C12 to C22 fatty acid or alkyl ester thereof, C12 to C22 alkane, C12 to C22 alkene, C12 to C22 alkyne, and C12 to C22 alcohol;
   at least one substrate selected from the group consisting of C12 to C18 fatty acid and alkyl ester thereof, triacylglycerol, diacylglycerol, and monoacylglycerol containing C12 to C18 fatty acid or alkyl ester thereof, fat or oil containing C12 to C18 fatty acid or alkyl ester thereof, C12 to C18 alkane, C12 to C18 alkene, C12 to C18 alkyne, and C12 to C18 alcohol; or
   at least one substrate selected from the group consisting of C12 to 18 fatty acid and alkyl ester thereof, triacylglycerol containing C12 to C18 fatty acid or alkyl ester thereof, and C12 to C18 alcohol.
[26] The method according to [25], wherein a content of the substrate in the medium is preferably 0.1 mass/volume% or more, more preferably 0.5 mass/volume% or more, further more preferably 1 mass/volume% or more, even more preferably 2 mass/volume% or more, even more preferably 3 mass/volume% or more, even more preferably 5 mass/volume% or more, and preferably 40 mass/volume% or less, more preferably 30 mass/volume% or less, further more preferably 25 mass/volume% or less, even more preferably 20 mass/volume% or less, even more preferably 15 mass/volume% or less, even more preferably 10 mass/volume% or less, or is preferably from 0.1 to 40 mass/volume%, from 0.1 to 30 mass/volume%, from 1 to 25 mass/volume%, from 0.1 to 20 mass/volume%, from 0.1 to 15 mass/volume%, from 0.1 to 10 mass/volume%, from 0.5 to 40 mass/volume%, from 0.5 to 30 mass/volume%, from 0.5 to 25 mass/volume%, from 0.5 to 20 mass/volume%, from 0.5 to 15 mass/volume%, from 0.5 to 10 mass/volume%, from 1 to 40 mass/volume%, from 1 to 30 mass/volume%, from 1 to 25 mass/volume%, from 1 to 20 mass/volume%, from 1 to 15 mass/volume%, from 1 to 10 mass/volume%, from 2 to 40 mass/volume%, from 2 to 30 mass/volume%, from 2 to 25 mass/volume%, from 2 to 20 mass/volume%, from 2 to 15 mass/volume%, from 2 to 10 mass/volume%, from 3 to 40 mass/volume%, from 3 to 30 mass/volume%, from 3 to 25 mass/volume%, from 3 to 20 mass/volume%, from 3 to 15 mass/volume%, from 3 to 10 mass/volume%, from 5 to 40 mass/volume%, from 5 to 30 mass/volume%, from 5 to 25 mass/volume%, from 5 to 20 mass/volume%, from 5 to 15 mass/volume%, or from 5 to 10 mass/volume%.
[27] The method according to any one of [23] to [26], further comprising recovering the glycolipid from the medium after the culturing.

### Examples

Hereinafter, the present invention will be described in more detail with reference to Examples. However, the technical scope of the present invention is not limited by the following Examples.

### Example 1

### (1) Extraction of genomic DNA from NBRC10243 strain

Genomic DNA was extracted and purified from *Starmerella bombicola* NBRC10243 strain (ATCC22214 strain) using Dr. GenTLE (for Yeast) High Recovery (Takara Bio Inc.) in accordance with the attached protocol.

### (2) Amplification of DNA fragment containing hygromycin resistance gene

The enzyme used for PCR performed below was PrimeSTAR Max DNA Polymerase (Takara Bio Inc.) in all cases unless otherwise specified. An expression cassette of hygromycin resistance gene was amplified with hygromycin resistance gene-containing DNA (SEQ ID NO: 3) prepared by artificial gene synthesis as a template using primers (SEQ ID NO: 4: GTTGTTGCTGGAGTCTCATCTGCAAG, and SEQ ID NO: 5: CCGGGTATACTAGTGATTTGAACAAAC) .

### (3) Amplification of 1 kbp upstream and 1 kbp downstream genomic DNA fragments of b0790 gene

With the genomic DNA of the NBRC10243 strain obtained in the section (1) of Example 1 as a template, 1 kbp downstream DNA fragment of b0790 gene consisting of the nucleotide sequence of SEQ ID NO: 1 was amplified using primers (SEQ ID NO: 6: GCCAAGCTTGCATGCTTGGGCACCTTCTGCGCACCCAGCACGGAG, and SEQ ID NO: 7: GACTCCAGCAACAACCATTAATATCGAGCTTGGCTCCTAATACTATAC) and 1 kbp upstream DNA fragment thereof was amplified using primers (SEQ ID NO: 8: CACTAGTATACCCGGACTTCGTATATGGCCAGGCTGCCTTGAG, and SEQ ID NO: 9: AGAGTCGACCTGCAGCAATGACGATAGCGAAGATCCCAC).

### (4) Amplification of vector fragment

A vector DNA fragment was amplified with pHSG298 (Takara Bio Inc.) as a template using primers (SEQ ID NO: 10: GCATGCAAGCTTGGCACTGGCCGTC, and SEQ ID NO: 11: CTGCAGGTCGACTCTAGAGGATCCCCG) .

### (5) Preparation of template plasmid pbJK9 of DNA fragment for introduction

Respective DNA fragments were purified from four PCR products described in the sections (2), (3), and (4) of Example 1 using NucleoSpin Gel and PCR Clean-up (Takara Bio Inc.) and then linked using In-Fusion HD cloning kit (Clontech Laboratories Inc.). Transformation into ECOS Competent E. Coli DH5α strain (Nippon Gene Co., Ltd.) was performed using the obtained plasmid solution, and the cell solution was applied to LB agar medium containing kanamycin and left standing overnight at 37°C. Colony PCR was performed with the obtained colony as a template using Sapphire Amp (Takara Bio Inc.) as an enzyme. The introduction of the DNA fragment of interest was confirmed using primers (SEQ ID NO: 12: CTCTTCGCTATTACGCCAGC, and SEQ ID NO: 13: CACTTTATGCTTCCGGCTCG). A transformant having the plasmid confirmed to have the introduced gene was inoculated to 2 mL of LB liquid medium containing kanamycin and cultured overnight at 37°C. The plasmid was purified from this culture solution using NucleoSpin Plasmid EasyPure (Takara Bio Inc.) to obtain a plasmid pbJK9 containing the DNA fragment in which the 1 kbp upstream fragment of the b0790 gene, the expression cassette of hygromycin resistance gene, and the 1 kbp downstream fragment of the b0790 gene were linked.

### (6) Preparation of DNA fragment for introduction

The DNA fragment in which the 1 kbp upstream fragment of the b0790 gene, the expression cassette of hygromycin resistance gene, and the 1 kbp downstream fragment of the b0790 gene were linked was amplified with the plasmid pbJK9 as a template using primers (SEQ ID NO: 14: TTGGGCACCTTCTGCGCACCCAG, and SEQ ID NO: 15: CAATGACGATAGCGAAGATCC). The obtained PCR product was treated with DpnI (Takara Bio Inc.), and the DNA fragment was further purified using NucleoSpin Gel and PCR Clean-up (Takara Bio Inc.).

### (7) Insertion of DNA fragment to NBRC10243 strain and preparation of Δb0790::hyg strain

Transformation into the NBRC10243 strain by electroporation (Nepa Gene Co., Ltd.) was performed using the DNA fragment obtained in the section (6) of Example 1 in which the 1 kbp upstream fragment of the b0790 gene, the expression cassette of hygromycin resistance gene, and the 1 kbp downstream fragment of the b0790 gene were linked. The cell solution after transformation was applied to YPD + hyg agar medium (1 mass/volume% yeast extracts, 2 mass/volume% tryptone, 2 mass/volume% glucose, 1.5 mass/volume% agar, 0.05 mass/volume% hygromycin) and left standing at 30°C for 2 days. PCR was performed with the obtained colony as a template using primers (SEQ ID NO: 16: CTTGACTGAGCGCTCAAGAAG, and SEQ ID NO: 17: GGTGCAGCCAACCACTAATTC), and the amplified fragment was subjected to sequence analysis. The introduced DNA fragment was confirmed to be introduced on the genome. This strain was designated as a Δb0790::hyge strain. This mutant strain had an insert of the expression cassette of hygromycin resistance gene so as to replace the b0790 gene on the genome.

### (8) Confirmation of glycolipid productivity of Δb0790::hyg strain

The Δb0790::hyg strain obtained in the section (7) of Example 1 and the NBRC10243 strain were separately inoculated to glycolipid production medium (0.1 mass/volume% urea, 2 mass/volume% yeast extracts, 5 mass/volume% oleic acid, 12.5 mass/volume% glucose) and shake-cultured under a condition of 30°C. 5 mL of the culture solution was recovered after culture for 72 hours, supplemented with 4 mL of hexane, and mixed by stirring for 5 seconds. After centrifugation for 5 minutes under conditions of 3 000 rpm and 25°C, a hexane fraction of a supernatant was removed. Subsequently, 6 mL of ethyl acetate was added to the remaining solution and mixed by stirring for 5 seconds. After centrifugation for 5 minutes under conditions of 3 000 rpm and 25°C, the whole amount of an ethyl acetate fraction was recovered. The recovered ethyl acetate fraction was volatilized by nitrogen gas blowing to deposit dissolved glycolipid (sophorolipid). The weight of the deposited sophorolipid was measured to calculate the concentration of the sophorolipid in the culture solution. The results are shown in Table 1. In the Δb0790::hyg strain, a sophorolipid concentration was increased by 32% as compared with the NBRC10243 strain, confirming an effect of improving sophorolipid productivity.

**[Table 1]**

| | Sophorolipid concentration after culture for 72 hours (g/L) |
|---|---|
| NBRC10243 strain | 44.94 |
| Δb0790::hyg strain | 59.14 |

### Example 2

### (1) Amplification of self-excising DNA fragment containing hygromycin resistance gene

The enzyme used for PCR performed below was PrimeSTAR Max DNA Polymerase (Takara Bio Inc.) in all cases unless otherwise specified. An expression cassette of hygromycin resistance gene was amplified with a self-excising expression cassette of hygromycin resistance gene: six-PgalK-βrec-Ttkt-Pgpd-HygR-Ttkt-six (SEQ ID NO: 18), as a template using primers (SEQ ID NO: 19: TCCGGAATTGACACATATAGGTCAATAGAGTATACTTATTTG, and SEQ ID NO: 20: TAAATCTAGATGATATATTATGCTCAACTTAAATGACCTACT) .

### (2) Amplification of vector fragment

A vector DNA fragment was amplified with pHSG298 (Takara Bio Inc.) as a template using primers (SEQ ID NO: 21: GCATGCAAGCTTGGCACTGGCCGTCG, and SEQ ID NO: 22: CTGCAGGTCGACTCTAGAGGATCCCC) .

### (3) Amplification of 1 kbp upstream and 1 kbp downstream genomic DNA fragments of fao1 gene

With the genomic DNA of the NBRC10243 strain obtained in the section (1) of Example 1 as a template, 1 kbp upstream DNA fragment of fao1 gene (SEQ ID NO: 33) was amplified using primers (SEQ ID NO: 23: GCCAAGCTTGCATGCGAAGGGGCTCTCCGAAGTACATCACTG, and SEQ ID NO: 24: TGTGTCAATTCCGGAAAAACGACAGAAAAGTGCTGAGGCCGC) and 1 kbp downstream DNA fragment thereof was amplified using primers (SEQ ID NO: 25: TATCATCTAGATTTATATCACAAGTCACTATTTACTTGTTTA, and SEQ ID NO: 26: AGAGTCGACCTGCAGCTCAATGAACTGAGACAGCAGCTTGTC).

### (4) Preparation of template plasmid pbJK27 of DNA fragment for introduction

Respective DNA fragments were purified from four PCR products described in the sections (1), (2), and (3) of Example 2 using NucleoSpin Gel and PCR Clean-up (Takara Bio Inc.) and then linked using In-Fusion HD cloning kit (Clontech Laboratories Inc.). Transformation into ECOS Competent E. Coli DH5α strain (Nippon Gene Co., Ltd.) was performed using the obtained plasmid solution, and the cell solution was applied to LB agar medium containing kanamycin and left standing overnight at 37°C. Colony PCR was performed with the obtained colony as a template using Sapphire Amp (Takara Bio Inc.) as an enzyme. The introduction of the DNA fragment of interest was confirmed using primers (SEQ ID NO: 27: CTCTTCGCTATTACGCCAGC, and SEQ ID NO: 28: CACTTTATGCTTCCGGCTCG). A transformant having the plasmid confirmed to have the introduced gene was inoculated to 2 mL of LB liquid medium containing kanamycin and cultured overnight at 37°C. The plasmid was purified from this culture solution using NucleoSpin Plasmid EasyPure (Takara Bio Inc.) to obtain a plasmid pbJK27 containing the DNA fragment in which the 1 kbp upstream fragment of the fao1 gene, the self-excising expression cassette of hygromycin resistance gene: six-PgalK-βrec-Ttkt-Pgpd-HygR-Ttkt-six, and the 1 kbp downstream fragment of the fao1 gene were linked.

### (5) Preparation of DNA fragment for introduction

The DNA fragment for fao1 gene disruption was amplified with the plasmid pbJK27 as a template using primers (SEQ ID NO: 29: GAAGGGGCTCTCCGAAGTACA, and SEQ ID NO: 30: CTCAATGAACTGAGACAGCAG). The obtained PCR product was treated with DpnI (Takara Bio Inc.), and the DNA fragment was further purified using NucleoSpin Gel and PCR Clean-up (Takara Bio Inc.).

### (6) Insertion of DNA fragment to NBRC10243 strain and preparation of Δfao1 strain

Transformation into the NBRC10243 strain by electroporation (Nepa Gene Co., Ltd.) was performed using the DNA fragment for fao1 gene disruption obtained in the section (5) of Example 2. The cell solution after transformation was applied to YPD + hyg agar medium (1 mass/volume% yeast extracts, 2 mass/volume% tryptone, 2 mass/volume% glucose, 1.5 mass/volume% agar, 0.05 mass/volume% hygromycin) and left standing at 30°C for 2 days. The obtained colony was inoculated to 5 mL of YNB + GAL liquid medium (0.67 mass/volume% Yeast Nitrogen Base w/o Amino Acids, 2 mass/volume% galactose) and shake-cultured at 30°C for 2 days. The culture solution was applied to YPD agar medium (1 mass/volume% yeast extracts, 2 mass/volume% tryptone, 2 mass/volume% glucose, 1.5 mass/volume% agar) and left standing at 30°C for 2 days. The obtained colony was applied to YPD agar medium and YPD + hyg agar medium, respectively. PCR was performed with the colony obtained only from the YPD agar medium as a template using primers (SEQ ID NO: 31: CTAAGGCCAGTACAAGAGATC, and SEQ ID NO: 32: CTAAAGCTCGAATTAATAGAGACACG), and the amplified fragment was subjected to sequence analysis. The fao1 gene was confirmed to be deleted. This strain was designated as a Δfao1 strain.

### (7) Insertion of DNA fragment to Δfao1 strain and preparation of Δfao1Δb0790::Hyg strain

Transformation into the Δfao1 strain by electroporation (Nepa Gene Co., Ltd.) was performed using the DNA fragment obtained in the section (6) of Example 1 in which the 1 kbp upstream fragment of the b0790 gene, the expression cassette of hygromycin resistance gene, and the 1 kbp downstream fragment of the b0790 gene were linked. The cell solution after transformation was applied to YPD + hyg agar medium (1 mass/volume% yeast extracts, 2 mass/volume% tryptone, 2 mass/volume% glucose, 1.5 mass/volume% agar, 0.05 mass/volume% hygromycin) and left standing at 30°C for 2 days. PCR was performed with the obtained colony as a template using primers (SEQ ID NO: 16 and SEQ ID NO: 17), and the amplified fragment was subjected to sequence analysis. The introduced DNA fragment was confirmed to be introduced on the genome. This strain was designated as a Δfao1Δb0790::hyg strain. This mutant strain had an insert of the expression cassette of hygromycin resistance gene so as to replace the b0790 gene on the genome.

### (8) Confirmation of glycolipid productivity of Δfao1 strain and Δfao1Δb0790::hyg strain

The Δfao1 strain and the Δfao1Δb0790::hyg strain were separately inoculated to glycolipid production medium (0.5 mass/volume% trisodium citrate dihydrate, 0.4 mass/volume% yeast extracts, 0.15 mass/volume% ammonium chloride, 0.07 mass/volume% magnesium sulfate heptahydrate, 0.05 mass/volume% sodium chloride, 0.027 mass/volume% calcium chloride dihydrate, 0.1 mass/volume% potassium dihydrogen phosphate, 0.016 mass/volume% dipotassium hydrogen phosphate, 15 mass/volume% glucose, 2.6mass/volume% oleyl alcohol) and shake-cultured under a condition of 30°C. 3 mL of the culture solution was recovered after culture for 4 days, supplemented with 3 mL of hexane, and mixed by stirring for 5 seconds. After centrifugation for 5 minutes under conditions of 3 000 rpm and 25°C, a hexane fraction of a supernatant was removed. Subsequently, 3 mL of butanol was added to the remaining solution and mixed by stirring for 5 seconds. After centrifugation for 5 minutes under conditions of 3 000 rpm and 25°C, the whole amount of a butanol fraction was recovered. Another 3 mL of butanol was added to the remaining solution, a similar process was repeated, and the whole amount of a butanol fraction was recovered. The recovered butanol fractions were volatilized by nitrogen gas blowing and the weight of precipitate which had dissolved was measured and defined as the concentration of Bola sophoroside in the culture solution. The results are shown in Table 2. In the Δfao1Δb0790::hyg strain, a Bola sophoroside concentration was increased to 1.96 times that in the Δfao1 strain, confirming an effect of improving Bola sophoroside productivity.

**[Table 2]**

| | Bola sophoroside concentration after culture for 4 days (g/L) |
|---|---|
| Δfao1 strain | 38.1 |
| Δfao1Δb0790::hyg strain | 74.6 |

## Claims

1. A yeast mutant strain in which expression of a polypeptide consisting of the amino acid sequence of SEQ ID NO: 2 or an amino acid sequence having at least 90% identity to the amino acid sequence of SEQ ID NO: 2 is suppressed or the polypeptide is inactivated.

2. The mutant strain according to claim 1, wherein a gene encoding the polypeptide consisting of the amino acid sequence of SEQ ID NO: 2 or an amino acid sequence having at least 90% identity to the amino acid sequence of SEQ ID NO: 2 is deleted or inactivated.

3. The mutant strain according to claim 2, wherein a gene consisting of the nucleotide sequence of SEQ ID NO: 1 or a gene which consists of a nucleotide sequence having at least 90% identity to the nucleotide sequence of SEQ ID NO: 1 and encodes the polypeptide consisting of the amino acid sequence of SEQ ID NO: 2 or an amino acid sequence having at least 90% identity to the amino acid sequence of SEQ ID NO: 2 is deleted or inactivated.

4. The mutant strain according to any one of claims 1 to 3, in which expression of a polypeptide consisting of the amino acid sequence of SEQ ID NO: 34 or an amino acid sequence having at least 90% identity to the amino acid sequence of SEQ ID NO: 34 is suppressed or the polypeptide is inactivated.

5. The mutant strain according to any one of claims 1 to 4, wherein the yeast is a yeast of the genus *Starmerella.*

6. A method for producing a yeast mutant strain, comprising suppressing expression of or inactivating a polypeptide consisting of the amino acid sequence of SEQ ID NO: 2 or an amino acid sequence having at least 90% identity to the amino acid sequence of SEQ ID NO: 2 in a yeast.

7. The method according to claim 6, comprising deleting or inactivating a gene encoding the polypeptide consisting of the amino acid sequence of SEQ ID NO: 2 or an amino acid sequence having at least 90% identity to the amino acid sequence of SEQ ID NO: 2.

8. The method according to claim 7, comprising deleting or inactivating a gene consisting of the nucleotide sequence of SEQ ID NO: 1 or a gene which consists of a nucleotide sequence having at least 90% identity to the nucleotide sequence of SEQ ID NO: 1 and encodes the polypeptide consisting of the amino acid sequence of SEQ ID NO: 2 or an amino acid sequence having at least 90% identity to the amino acid sequence of SEQ ID NO: 2.

9. The method according to any one of claims 6 to 8, wherein the yeast is a yeast of the genus *Starmerella.*

10. A method for improving ability of a yeast to produce a glycolipid, comprising suppressing expression of or inactivating a polypeptide consisting of the amino acid sequence of SEQ ID NO: 2 or an amino acid sequence having at least 90% identity to the amino acid sequence of SEQ ID NO: 2 in the yeast.

11. The method according to claim 10, comprising deleting or inactivating a gene encoding the polypeptide consisting of the amino acid sequence of SEQ ID NO: 2 or an amino acid sequence having at least 90% identity to the amino acid sequence of SEQ ID NO: 2.

12. The method according to claim 11, comprising deleting or inactivating a gene consisting of the nucleotide sequence of SEQ ID NO: 1 or a gene which consists of a nucleotide sequence having at least 90% identity to the nucleotide sequence of SEQ ID NO: 1 and encodes the polypeptide consisting of the amino acid sequence of SEQ ID NO: 2 or an amino acid sequence having at least 90% identity to the amino acid sequence of SEQ ID NO: 2.

13. The method according to any one of claims 10 to 12, wherein the yeast is a yeast of the genus *Starmerella.*

14. The method according to any one of claims 10 to 13, wherein the glycolipid is selected from sophorolipid and Bola sophoroside.

15. A method for producing a glycolipid, comprising culturing the yeast mutant strain according to any one of claims 1 to 5.

16. The method according to claim 15, wherein the glycolipid is selected from sophorolipid and Bola sophoroside.
